# EUROPÄISCHE PATENTSCHRIFT

(11) **EP 2 116 497 B1**
(45) Veröffentlichungstag und Bekanntmachung des Hinweises auf die Patenterteilung: **10.02.2016**
(21) Anmeldenummer: 09005979.1
(22) Anmeldetag: 30.04.2009
(51) Int. Cl.: A61M 25/00, B65H 75/44

(54) **Einrichtung zur Aufnahme eines flexiblen medizinischen Führungsdrahtes**
Device for holding a flexible medical guidewire
Dispositif de réception d'un fil de guidage médical flexible

(30) Priorität: 07.05.2008 DE 102008022666
(43) Veröffentlichungstag der Anmeldung: 11.11.2009
(73) Patentinhaber: Endox Feinwerktechnik GmbH, 72574 Bad Urach (DE)
(72) Erfinder: Hernik, Matthias, 72574 Bad Urach (DE)
(74) Vertreter: Lohr, Georg

(56) Entgegenhaltungen:
- EP-A1- 0 050 606
- WO-A1-97/11736
- WO-A1-2007/081264
- DE-A1- 2 845 629
- DE-C- 705 802
- GB-A- 2 215 703
- US-A- 2 711 734
- US-A- 6 086 008
- US-A1- 2006 264 921

## Beschreibung

Die Erfindung betrifft eine Einrichtung zur Aufnahme eines flexiblen medizinischen Führungsdrahtes mit einer Wickelgutaufnahme und einem die Wickelgutaufnahme zumindest abschnittsweise abdeckenden Gehäuse, die einen Aufnahmeraum zur Aufnahme des Wickelgutes bilden.

Aus der US 6,086,008 ist eine Wickelvorrichtung bekannt, welche eine in ein Gehäuseteil einsetzbare Wickelgutaufnahme aufweist, die einen Aufnahmeraum zur Aufnahme des Wickelgutes aufweist. Die Wickelgutaufnahme und das Gehäuseteil sind relativ zueinander verdrehbar miteinander verbunden, um das Wickelgut aufzuwickeln. Nach dem Aufwickeln wird die Wickelgutaufnahme aus dem Gehäuseteil abgehoben und entfernt.

Aus der WO 2007/081264 A1 geht eine Aufnahmevorrichtung für einen Katheter hervor, die ein zweiteiliges Gehäuse umfasst, in welches ein in einer Hülse drehbar gelagerter Wickelkern einsetzbar ist. Das zweiteilige Gehäuse weist zumindest einen öffenbaren Gehäusedeckel auf, durch welchen der Innenraum des Gehäuses zugänglich ist.

Aus der EP 0 050 606 A1 geht eine Abwickelvorrichtung für einen Katheter hervor, die eine Anzeige umfasst, um die abgezogene Länge des Katheters abzulesen. Dafür ist in dem Deckel ein Fenster und ein Getriebe vorgesehen. Der Deckel wird auf einer Wickelgutaufnahme positioniert, welche einen separaten Aufnahmeraum aufweist. Bei dieser Abwickelvorrichtung ist vorgesehen, dass vor dem Verschließen der Wickelgutaufnahme der Katheter in den Aufnahmeraum manuell eingelegt wird, damit anschließend nur noch ein Abziehen erfolgt.

Aus der GB 2 215 703 A ist des Weiteren ein Dispenser für einen Führungsdraht bekannt, bei dem eine Wickelgutaufnahme einen Aufnahmeraum für einen Katheter umfasst, der durch einen Deckel verschlossen ist. Über eine im Außenumfang zwischen der Wickelgutaufnahme und dem Deckel gebildete Öffnung wird der Führungsdraht nach außen geführt. Nachdem der Führungsdraht in den Aufnahmeraum der Wickelgutaufnahme manuell eingelegt ist, wird die Wickelgutaufnahme durch den Deckel verschlossen, wobei dieser feststehend zur Wickelgutaufnahme angeordnet ist.

Aus der DE 197 51 194 C1 ist ein Drahtdispenser für elastisch verformbare Drähte bekannt, der ein sich über einen Umfangswinkel von mehr als 180° erstreckendes Ringelement umfasst, in das ein elastisch verformbarer Draht gewickelt einlegbar ist. Zum Einbringen des elastisch verformbaren Drahtes weist ein solches Ringelement ein offenes Querschnittsprofil mit einem in Umfangsrichtung längsverlaufenden durchgehenden Drahteinlegeschlitz auf, der radial innenliegend ausgebildet ist. Dieser radial innenliegende Drahteinlegeschlitz wird durch ein in dem Ringelement lösbar befestigbares Abdeckelement wenigstens abschnittsweise abgedeckt, um den elastisch verformbaren Draht in dem Ringelement zu sichern. Eine analoge Anordnung ist bei einem Drahteinlegeschlitz vorgesehen, der in einer Seitenflanke des Ringelementes vorgesehen ist.

Solche Drahtdispenser sind dafür vorgesehen, um elastisch verformbare Drähte zu bevorraten, welche in der Medizin, beispielsweise in der Katheterisierung, eingesetzt werden. Solche Führungsdrähte weisen beispielsweise eine Länge von vier Metern auf. Damit eine sichere und einfache Handhabung der sehr dünnen Drähte ermöglicht ist, werden diese in solchen Drahtdispensern bevorratet.

Die aus der DE 197 51 194 C1 bekannte Vorrichtung weist jedoch den Nachteil auf, dass beim Einbringen des Drahtes in einen Aufnahmeraum des Ringelementes, welches einen C-förmigen Querschnitt aufweist, die einzelnen Windungen in beliebiger Weise in dem Aufnahmeraum des Ringelementes sich anordnen. Dadurch kann insbesondere beim schnellen Abziehen des Drahtes aus dem Drahtdispenser die Gefahr des sich Verhedderns bestehen, wodurch ein weiteres Abziehen nicht mehr möglich ist oder einen erheblichen Zeitaufwand erfordert.

Der Erfindung liegt deshalb das technische Problem zugrunde, eine Einrichtung zu schaffen, der zur Wiederverwendung nach dem erstmaligen Gebrauch eines medizinischen Führungsdrahts in einfacher Weise wiederbefüllbar und anschließend für die Weiterverwendung sicher und schnell zu entnehmen ist.

Diese Aufgabe wird erfindungsgemäß durch die Merkmale des Anspruchs 1 gelöst.

Die erfindungsgemäße Einrichtung, insbesondere für flexible medizinische Instrumente, weist in dem Aufnahmeraum eine Wickelgutführung auf, welche beim Aufwickeln des Wickelgutes mehrere Windungen des Wickelgutes nebeneinander anordnet. Die Wickelführung ist durch eine profilierte Oberfläche in dem Aufnahmeraum ausgebildet. Diese profilierte Oberfläche weist zur Bildung der Wickelgutführung eine umlaufende Rille auf, die durch einen dazwischen angeordneten Steg separiert ist, welcher sich schneckenförmig fortsetzt, so dass die Windungen des Führungsdrahtes nebeneinander in einer Ebene im Aufnahmeraum angeordnet und jeweils getrennt voneinander aufgewickelt sind.

Dadurch wird während dem Aufwickeln des Wickelgutes beziehungsweise der Aufnahme des Wickelgutes in dem Aufnahmeraum der Einrichtung eine Windung neben die andere gelegt, so dass eine geordnete Wickelanordnung vorgesehen ist, wodurch eine schnelle Entnahme des Wickelgutes ermöglicht ist, da eine Wicklung nach der anderen abgezogen werden kann, ohne dass eine benachbarte Windung beeinflusst wird. Eine solche Wickelaufnahme, welche jede Windung benachbart zur weiteren Windung anordnet, kann aufgrund der Ausgestaltung der Wickelgutführung, in einfacher Weise bedient werden, so dass ein Befüllen der Einrichtung sichergestellt ist und die einzelnen Windungen nebeneinander in dem Aufnahmeraum angeordnet werden. Zur einfachen Handhabung der Einrichtung ist an einer Außenseite der Wickelgutaufnahme und/oder an der Außenseite der Grundfläche zumindest eine Grifffläche oder Griffmulde vorgesehen. Somit kann eine sichere Betätigung ermöglicht sein. Die Einrichtung ist für medizinische Führungsdrähte ausgebildet.

Nach einer bevorzugten Ausführungsform der Erfindung ist vorgesehen, dass die Wickelgutführung durch eine gerillte Oberfläche in dem Aufnahmeraum ausgebildet ist.

Eine bevorzugte Ausgestaltung der Erfindung sieht vor, dass ein Abstand zwischen einer Anlagefläche des Gehäuseteils und einer Stirnfläche der umlaufenden Rille kleiner als der Durchmesser, insbesondere der halbe Durchmesser, des kleinsten aufzunehmenden Wickelgutes ausgebildet ist. Dadurch wird sichergestellt, dass die einzelnen Windungen in den durch die umlaufende Rille gebildeten Einzelkammern nicht aneinander liegen. Darüber hinaus wird der Vorteil erzielt, dass jede Windung vollständig in der umlaufenden Rille aufgenommen werden kann.

Nach einer weiteren bevorzugten Ausgestaltung der Erfindung ist vorgesehen, dass eine wirksame Höhe des Aufnahmeraumes kleiner als der doppelte Durchmesser des Wickelgutes ausgebildet ist. Die wirksame Höhe umfasst sowohl die Rillentiefe als auch den Abstand zwischen der Stirnseite der Wickelaufnahme und der gegenüberliegenden Anlagefläche am Gehäuseteil. Dadurch wird sichergestellt, dass die nebeneinander liegenden Windungen des Wickelgutes in dem Aufnahmeraum gesichert zur profilierten Oberfläche liegen, so dass ein Verheddern oder Verschränken von einzelnen Windungen verhindert ist.

Zum einfachen Ein- und Ausfädeln des Wickelgutes ist bevorzugt vorgesehen, dass die Wickelgutaufnahme und das Gehäuseteil, welche gemeinsam den Aufnahmeraum bilden beziehungsweise umgeben, relativ zueinander verdrehbar angeordnet sind. Somit kann durch Verdrehen des einen oder anderen Teils oder einem gleichzeitigen Verdrehen beider Teile der Einrichtung ein schneller Aufwickel- oder Abwickelvorgang erzielt werden.

An dem Gehäuseteil ist eine zum Aufnahmeraum führende Einführöffnung vorgesehen. Dadurch kann ein gezieltes Zuführen und Herausführen des Wickelgutes gegeben sein. Bevorzugt erstreckt sich die Einführöffnung über den gesamten Aufnahmeraum, so dass gleichzeitig eine Sichtkontrolle bezüglich des bereits aufgewickelten oder sich noch in der Einrichtung befindenden Wickelgutes ermöglicht ist.

Die Einführöffnung im Gehäuseteil weist bevorzugt eine Einführschräge auf, welche zum Aufnahmeraum hin ausgerichtet ist. Dadurch kann ein einfaches Einführen des Wickelgutes ohne Knickung in den Aufnahmeraum gegeben sein.

Nach einer weiteren bevorzugten Ausgestaltung der Erfindung ist vorgesehen, dass die Wickelgutführung zum Aufnahmeraum, welche insbesondere als gerillte Oberfläche ausgebildet ist, eine Rillentiefe aufweist, die wenigstens die Hälfte des Wickelgutdurchmessers umfasst. Dadurch wird ermöglicht, dass jede Windung des Wickelgutes sicher in der Rille aufgenommen und darin gehalten ist. Die benachbarten Windungen werden getrennt voneinander angeordnet. Somit können diese sich während dem Auf- und Abwickeln nicht beeinflussen.

Der Aufnahmeraum, der die nebeneinander angeordneten Windungen des Wickelgutes aufnimmt, ist gemäß einer ersten vorteilhaften Ausführungsform der Erfindung radial zur Rotationsachse der Wickelgutaufnahme und des Gehäuseteils ausgerichtet. Insbesondere ist die profilierte Oberfläche der Wickelgutaufnahme radial zur Rotationsachse des Gehäuseteils und der Wickelgutaufnahme ausgebildet. Eine solche Ausführungsform weist den Vorteil auf, dass eine scheibenförmige, insbesondere flach scheibenförmige, Einrichtung geschaffen ist, der ein geringes Aufbauvolumen umfasst.

Der Aufnahmeraum der Einrichtung weist bevorzugt Nocken auf, die der profilierten Oberfläche gegenüberliegend angeordnet sind und sich vorzugsweise quer zur profilierten Oberfläche erstrecken. Durch diese Nocken wird erzielt, dass die Anlagefläche des in dem Aufnahmeraum sich befindenden Wickelgutes erheblich minimiert wird. Das Wickelgut liegt unmittelbar auf der profilierten Oberfläche auf und wird durch die Drehung der Wickelgutaufnahme oder des Gehäuseteiles in einer ruhenden Position gehalten. Die Nocken im Aufnahmeraum liegen der profilierten Oberfläche gegenüber. Dadurch kann die Handhabung beim Auf- und Abwickeln mit einer geringeren Kraft und verringerten Reibung erfolgen.

Die Wickelgutaufnahme und das Gehäuseteil, welche die Einrichtung bilden, sind bevorzugt durch eine lösbare Rastverbindung miteinander verbunden. Dadurch können diese zum Autoklavieren in einfacher Weise und schnell zerlegt und voneinander getrennt werden. Aufgrund der bevorzugten zweiteiligen Ausführungsform der Einrichtung ist nach dem Autoklavieren ein einfacher und schneller Zusammenbau ohne zusätzliche Hilfsinstrumente möglich.

In zumindest einem der beiden Teile der Einrichtung ist zumindest ein federnd nachgiebiges Rastelement vorgesehen, welches an dem anderen Teil angreift und die lösbare Rastverbindung bildet, so dass die beiden Teile einander fest zugeordnet, jedoch relativ zueinander verdrehbar angeordnet sind. Beispielsweise können hierfür Rasthaken vorgesehen sein, die an einer gegenüberliegenden Führungsfläche des anderen Teils angreifen. Ebenso können Rastnasen vorgesehen sein, die in einer Vertiefung des anderen Teils eingreifen und gleichzeitig eine Drehbewegung in der Wickelgutaufnahme relativ zum Gehäuseteil ermöglichen.

Des Weiteren ist bevorzugt an dem Gehäuseteil oder der Wickelgutaufnahme ein Lueranschluss oder eine Spülöffnung vorgesehen. Durch einen solchen Lueranschluss kann in einfacher Weise eine Spülung des Aufnahmeraumes erfolgen. Dadurch kann gleichzeitig eine Reinigung des Wickelgutes ermöglicht werden.

Die Erfindung sowie weitere vorteilhafte Ausführungsformen und Weiterbildungen derselben werden im Folgenden anhand der in den Zeichnungen dargestellten Beispiele näher beschrieben und erläutert. Es zeigen:
- Figur 1: eine erste Seitenansicht einer ersten Ausführungsform der erfindungsgemäßen Einrichtung,
- Figur 2: eine weitere Seitenansicht der Einrichtung gemäß Figur 1,
- Figur 3: eine schematische Schnittdarstellung der Einrichtung gemäß Figur 1,
- Figur 4: eine schematische Ansicht auf ein Gehäuseteil der Einrichtung gemäß Figur 1 und
- Figur 5: eine schematisch vergrößerte Ansicht einer Einführöffnung am Gehäuseteil der Einrichtung gemäß Figur 1.

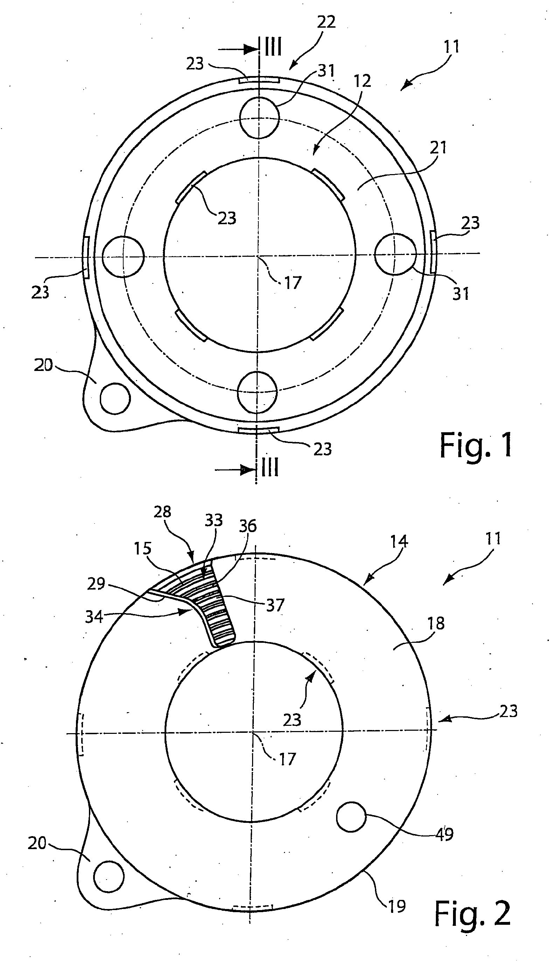

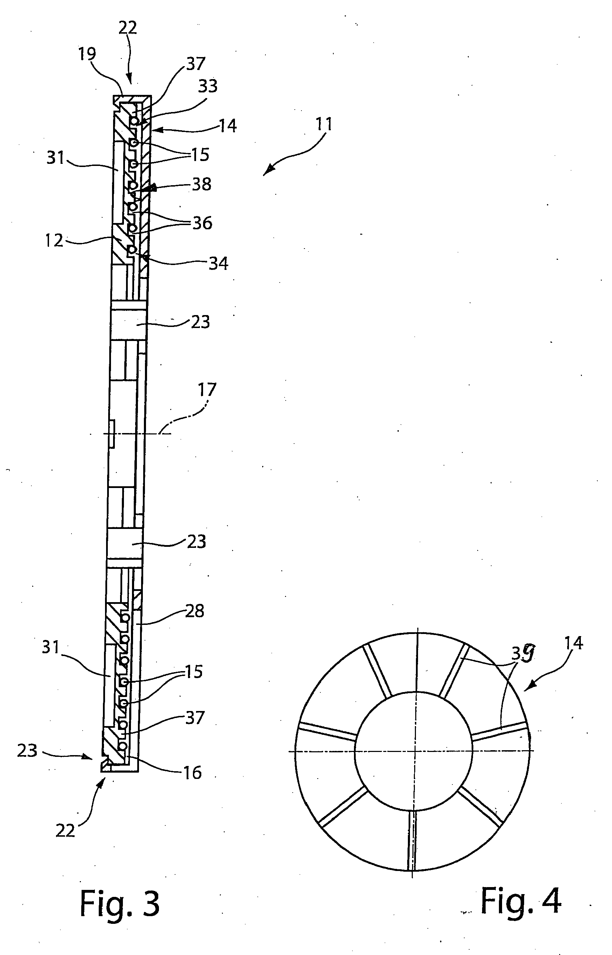

In Figur 1 ist eine erste Seitenansicht auf eine erfindungsgemäße Einrichtung 11 dargestellt. Die Figur 2 zeigt eine gegenüberliegende Seite der Einrichtung 11 in Figur 1. In Figur 3 ist ein schematischer Vollschnitt entlang der Linie III-III in Figur 1 dargestellt.

Die Einrichtung 11 besteht bevorzugt nur aus zwei Teilen und umfasst eine Wickelgutaufnahme 12 sowie ein die Wickelgutaufnahme 12 zumindest abschnittsweise umgebendes beziehungsweise zumindest abschnittsweise abdeckendes Gehäuseteil 14. Zwischen der Wickelgutaufnahme 12 und dem Gehäuseteil 14 ist ein ringförmiger Aufnahmeraum 16 ausgebildet, der zur Aufnahme eines elastisch verformbaren Wickelgutes 15, insbesondere von elastisch verformbaren Instrumenten, wie beispielsweise eines medizinischen Führungsdrahtes, eine Schlinge, Biopsiezange oder eines Katheders oder dergleichen, vorgesehen ist. Bevorzugt findet diese Einrichtung als Medizininstrumentedispenser seinen Einsatz.

Das Gehäuseteil 14 und die Wickelgutaufnahme 12 sind relativ zueinander verdrehbar um die Rotationsachse 17 zueinander angeordnet. Das Gehäuseteil 14 weist hierzu eine ringförmige Grundfläche 18 mit einer äußeren radial sich erstreckenden Mantelfläche 19 auf, innerhalb der die Wickelgutaufnahme 12, welche bevorzugt als scheibenförmiges Ringelement 21 ausgebildet ist, anordenbar ist. Die Wickelgutaufnahme 12 und das Gehäuseteil 14 sind durch eine lösbare Rastverbindung 22 miteinander verbunden. Die Mantelfläche 19 des Gehäuseteils 14 umfasst zumindest ein Rastelement 23, um die Wickelgutaufnahme 12 lösbar zum Gehäuseteil 14 anzuordnen. Das Rastelement 23 ist beispielsweise als Rasthaken oder Hintergreifung ausgebildet, der einteilig an der Grundfläche 18 des Gehäuseteils 14 angeformt ist. Diese können bevorzugt an einer äußeren Schulter oder Wickelaufnahme angreifen und das Gehäuseteil 14 zur Wickelaufnahme 12 lagefixieren. Damit das Rastelement 23 federnd nachgiebig ausgebildet ist, können beispielsweise U-förmige Ausnehmungen in der Grundfläche 18 des Gehäuseteils 14 vorgesehen sein. Solche Rastelemente 23 können an einer äußeren Mantelfläche 19 vorgesehen sein. Diese Rastelemente 23 können auch an einem Innenumfang des scheibenförmigen Gehäuseteils 14 angeordnet werden. Zusätzlich kann der Innenumfang eine weitere Mantelfläche in Analogie zur äußeren Mantelfläche 19 umfassen, wobei die Rastelemente 23 am Innenumfang einen Teil dieser Mantelfläche bilden können. An dem Gehäuseteil 14 ist bevorzugt eine Haltelasche 20 vorgesehen.

In der Grundfläche 18 des Gehäuseteils 14 ist eine Einführöffnung 28 vorgesehen, welche sich vorzugsweise in radialer Richtung über die gesamte Breite des Aufnahmeraumes 16 erstreckt. An einer Längskante der Einführöffnung 28 ist eine Einführschräge 29 vorgesehen, welche die Dicke der Wandstärke der Grundfläche 18 zum Aufnahmeraum 16 hin verjüngt. Dadurch kann das Auf- und Abwickeln des Wickelgutes 15 knickungsfrei und vereinfacht erfolgen.

An einer Außenseite der Wickelgutaufnahme 12 ist zumindest eine Grifffläche 31, insbesondere eine Griffmulde, vorgesehen. Solche Griffflächen 31 können ebenfalls an der Außenseite der Grundfläche 18 vorgesehen sein.

Die Griffflächen 31, insbesondere Griffmulden, können mit einem fluoreszierenden Material oder einer fluoreszierenden Beschichtung ausgebildet sein. Dadurch wird ermöglicht, dass eine einfache Handhabung der Einrichtung 11 auch bei abgedunkelten Operationsräumen möglich ist. Des Weiteren kann bevorzugt vorgesehen sein, dass ergänzend die Körperkanten der Einführöffnung 28, insbesondere die Einführschräge, ebenfalls aus fluoreszierendem Material besteht oder mit einer fluoreszierenden Beschichtung versehen ist, um den Zuführbereich des Wickelgutes 15 zu dessen Aufnahme als auch den Entnahmebereich zu dessen Entnahme zu kennzeichnen.

Der Aufnahmeraum 16 der Einrichtung 11 umfasst eine Wickelgutführung 33, die eine Zwangsführung bei dem Aufwickel- und Abwickelvorgang des Wickelgutes 15 ermöglicht. Diese Wickelgutführung 33 wird durch eine profilierte Oberfläche 34 in der Wickelgutaufnahme 12 gebildet, welche in den Aufnahmeraum 16 weist. Diese profilierte Oberfläche 34 ist insbesondere als gerillte Oberfläche 34 ausgebildet und besteht aus einer umlaufenden Rille 36, wobei beispielsweise am Innenumfang ein Rillenende (nicht näher dargestellt) vorgesehen ist, welches den Wickelbeginn zum Aufwickeln des Wickelgutes 15 bildet. Alternativ kann ein Rillenende auch am Außenumfang der Wickelgutführung 33 vorgesehen sein. In Abhängigkeit der Länge des aufzuwickelnden Wickelgutes 15 können eine oder mehrere Rillen 36 beziehungsweise Windungen der Wickelgutführung 33 gelegt werden. Aufgrund der Einführöffnung 28 kann durch Drehen des Gehäuseteils 14 und/oder der Wickelgutaufnahme 12 jeweils um wenigstens 180° ein Ende des Wickelgutes 15 erfasst werden, welches den Beginn zum Abwickeln des Wickelgutes darstellt. Die Positionierung des Wickelendes des Wickelgutes kann beispielsweise durch eine Markierung an der Eintrittsöffnung 28 oder durch eine verlängerte Vertiefung an der Eintrittsöffnung 28 vorgesehen sein, wobei die verlängerte Öffnung oder Vertiefung in der Kontur an die benachbarte Rille 36 angepasst ist.

Die Rille 36 ist bevorzugt durch dazwischen angeordnete Stege 37 separiert, so dass die einzelnen Windungen des Wickelgutes 15 nebeneinander in einer Ebene im Aufnahmeraum 16 angeordnet und jeweils getrennt zueinander separiert vorgesehen sind. Die Höhe der Stege 37 ist gemäß einer bevorzugten Ausführungsform der Erfindung derart bemessen, dass der Durchmesserbereich der aufzunehmenden Drähte vollständig in einem zwischen den Stegen 37 und einem Rillenboden gebildeten Raum aufgenommen werden können. Dadurch kann ein Abstand zwischen einer Stirnseite des Steges 37 und der gegenüberliegenden Anlagefläche 38 des Gehäuseteils 14 sehr gering ausgebildet werden. Dies weist den Vorteil auf, dass bei der Aufnahme von sehr dünnen Drähten ein Verklemmen oder ein Verhaken in diesem Bereich verhindert wird. Der Abstand zwischen der Stirnseite des Steges 37 und der Anlagefläche 38 des Gehäuseteils 14 ist bevorzugt kleiner als der kleinste Durchmesser des aufzunehmenden Wickelgutes 15 ausgebildet. Durch eine solche Ausgestaltung ist ermöglicht, dass das in dem Aufnahmeraum 16 aufgenommene Wickelgut 15 vollständig innerhalb dem Freiraum zwischen den Stegen 37 angeordnet ist, so dass eine Anlage des aufgewickelten Wickelgutes 15 an der Anlagefläche 38 des Gehäuseteils 14 verhindert oder weitestgehend verhindert ist. Durch die Eigenspannung des Wickelgutes 15 liegt dieser jeweils an einer den Außenumfang des jeweiligen Hohlraumes begrenzenden Stirnfläche des Steges an.

Nach einer weiteren bevorzugten Ausgestaltung der Einrichtung 11 ist vorgesehen, dass benachbart zur Einführöffnung 28 auf einer Außenseite des Gehäuseteils 14 Markierungen vorgesehen sind, welche von radial außen nach radial innen entlang der Einführöffnung 28 vorgesehen sind. Diese Markierungen können mit unterschiedlichen Färbungen und/oder zusätzlich mit Zahlenangaben versehen sein. Bevorzugt umfassen die Zahlenangaben Längenangaben, die dem aufgewickelten Wickelgut 15 entsprechen. Dadurch wird ermöglicht, dass zum einen der Bereich für den Benutzer gekennzeichnet wird, der den Beginn einer Aufwickelstelle bei vorbestimmter Länge des Wickelgutes 15, insbesondere des Führungsdrahtes, aufweist. Darüber hinaus weisen diese Markierungen den Vorteil auf, dass bei beispielsweise einem vollständig sich über den Aufnahmeraum 16 entlang den Rillen 36 erstreckenden Wickelgut 15 nach der teilweisen Entnahme des Wickelgutes aufgrund der Markierung ein Rückschluss auf die entnommene Menge bzw. Länge des Wickelgutes 15 ermöglicht wird. Somit können solche Markierungen einen Doppelnutzen aufweisen.

Nach einer weiteren nicht näher dargestellten alternativen Ausführungsform kann vorgesehen sein, dass die Höhe der Stege 37 wenigstens die Hälfte des Durchmessers des Wickelgutes umfasst. Die Höhe des gesamten Aufnahmeraumes 16, also vom Rillenboden bis zur gegenüberliegenden Anlagefläche 38, die am Gehäuseteil 14 vorgesehen ist, beträgt weniger als der doppelte Durchmesser des Wickelgutes. Die Anlagefläche 38 kann bevorzugt auch durch halbrunde Nocken 39 ausgebildet sein, welche sich bei einem radial zur Rotationsachse der Einrichtung 11 ausgerichteten Aufnahmeraums 16 sternförmig nach außen erstrecken. Dies geht beispielsweise aus Figur 4 hervor. Durch diese Nocken 39 wird die Größe der Anlagefläche 38 des in dem Aufnahmeraum 16 sich befindenden Wickelgutes 15 und die Reibung des Gehäuseteils 14 reduziert, wodurch die Reibung verringert wird und das Auf- und Abwickeln erleichtert ist.

Die Wickelgutaufnahme 12 und das Gehäuseteil 14 sind bevorzugt als Spritzgussteil aus Kunststoff ausgebildet. Sofern die Einrichtung 11 mehrfach eingesetzt werden soll, wird ein Kunststoff verwendet, der autoklavierbar ist. Aufgrund der nur zweiteiligen Ausgestaltung der Einrichtung 11 ist ein einfaches Zerlegen und Zusammenbauen in kurzer Zeit ermöglicht, wodurch auch die Reinigung erleichtert sein kann.

An dem Gehäuseteil 14 ist beispielsweise eine Spülöffnung 49 vorgesehen, welche ermöglicht, dass eine Reinigungs- oder Spülflüssigkeit oder Gleitflüssigkeit dem Aufnahmeraum 16 zugeführt werden kann, welche sich innerhalb des Aufnahmeraumes 16 verteilt und über die Einführöffnung 28 oder über die radial außen liegende Schnittstelle zwischen der Wickelaufnahme 12 und dem Gehäuseteil 14 austritt. Ebenso kann es sich um einen sogenannte Luer-Anschluss handeln.

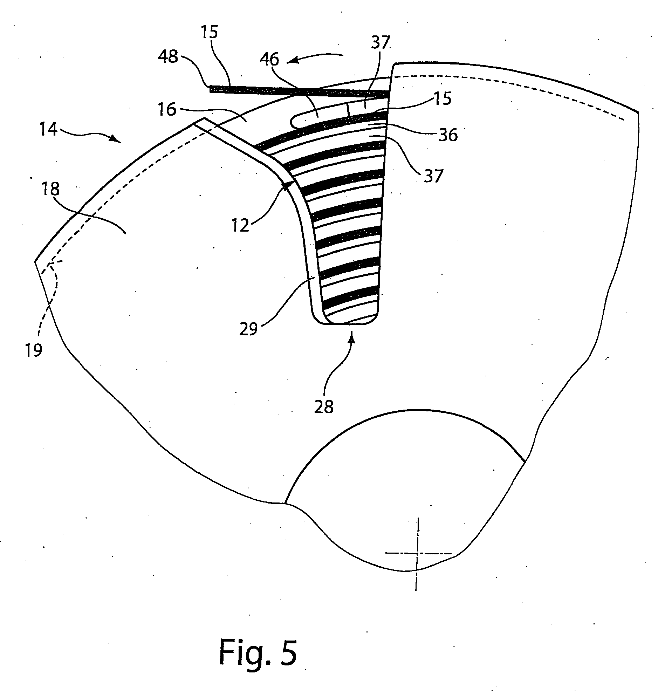

In Figur 5 ist schematisch vergrößert die Einführöffnung 28 dargestellt. Diese Einführöffnung 28 erstreckt sich nicht nur radial entlang des Aufnahmeraumes 16, wie in Figur 5 dargestellt ist, sondern auch axial zumindest über die Höhe der Stege 37. Darüber hinaus ist bevorzugt vorgesehen, dass der radial äußere Bereich der Austrittsöffnung 28 breiter als der radial innere Bereich ausgebildet ist. Die zunehmende Öffnungsbreite kann, wie im Ausführungsbeispiel dargestellt ist, durch einen diskontinuierlichen Verlauf vorgesehen sein oder aber auch einen kontinuierlichen gekrümmten Verlauf umfassen. Der Aufnahmeraum 16 weist bevorzugt am äußersten Ende der Rille einen Anfangsabschnitt 46 auf, der sich vom Rillenboden aus kontinuierlich bis zur Höhe des Steges 37 erstreckt. Der Steg 37 setzt sich anschließend schneckenförmig fort, um den Aufnahmeraum 16 zu bilden. Beim Abwickeln beziehungsweise bei der Entnahme des Wickelgutes 15 erstreckt sich ein freies Ende 48 des Wickelgutes 15 aufgrund der Eigenspannung aus der Einführöffnung 28 radial nach außen. Beim weiteren Verdrehen des Gehäuseteils 14 und der Wickelgutaufnahme 12 wird das Wickelgut 15 aus dem Aufnahmeraum 16 entlang der Einführschräge 29 herausgeführt. Zunächst wird eine äußere Windung dem Aufnahmeraum 16 entnommen. Diese äußere Windung liegt in einem zwischen dem Steg 37 und einer Mantelfläche 19 des Gehäuseteils 18 gebildeten Raum. Nachdem diese Windung vollständig entnommen ist, folgt das Wickelgut 15 entlang des Anfangsabschnittes 46, der stetig ansteigt, so dass eine gesicherte Wickelgutherausführung beim Übergang von einem freien Raum zwischen dem Steg 37 und der Mantelfläche 19 in einen Raum zwischen zwei benachbarten Stegen 37 sichergestellt ist.

## Patentansprüche

1. Einrichtung zur Aufnahme eines flexiblen medizinischen Führungsdrahtes (15),
mit einer Wickelgutaufnahme (12) und
mit einem die Wickelgutaufnahme (12) zumindest abschnittsweise abdeckenden Gehäuseteil (14), die einen ringförmigen Aufnahmeraum (16) zur Aufnahme des medizinischen Führungsdrahtes (15) bilden,
wobei in der Wickelgutaufnahme (12) eine Wickelgutführung (33) vorgesehen ist, welche beim Aufwickeln des Führungsdrahtes (15) mehrere Windungen des Führungsdrahtes (15) nebeneinander anordnet,
wobei die Wickelgutführung (33) durch eine profilierte Oberfläche in dem Aufnahmeraum (16) ausgebildet ist und die Oberfläche (34) aus einer umlaufenden Rille (36) gebildet ist, die durch einen dazwischen angeordneten Steg (37) separiert ist, welcher sich schneckenförmig fortsetzt, so dass die Windungen des Führungsdrahtes (15) nebeneinander in einer Ebene im Aufnahmeraum (16) angeordnet und jeweils getrennt voneinander aufgewickelt sind,
**dadurch gekennzeichnet,**
**dass** die Wickelgutaufnahme (12) und das Gehäuseteil (14) um eine gemeinsame Rotationsachse (17) relativ zueinander verdrehbar miteinander verbunden sind, dass in dem Gehäuseteil (14) eine zum Aufnahmeraum (16) führende Einführöffnung (28) vorgesehen ist, und
**dass** an einer Außenseite der Wickelgutaufnahme (12) und/oder an einer Außenseite einer Grundfläche (18) des Gehäuseteils (14) zumindest eine Grifffläche (31), insbesondere eine Griffmulde, vorgesehen ist.

2. Einrichtung nach Anspruch 1,
**dadurch gekennzeichnet, dass**
die umlaufende Rille (36) eine Rillentiefe aufweist, welche wenigstens dem halben Durchmesser des aufzunehmenden Führungsdrahtes (15) entspricht.

3. Einrichtung nach Anspruch 1,
**dadurch gekennzeichnet, dass**
ein Abstand zwischen einer Anlagefläche (38) des Gehäuseteils (14) und einer Stirnfläche der umlaufenden Rille (36) kleiner als der im Durchmesser kleinste aufzunehmende Führungsdraht (15),insbesondere kleiner als der halbe Durchmesser des kleinsten aufzunehmenden Führungsdrahtes (15),ausgebildet ist.

4. Einrichtung nach Anspruch 1,
**dadurch gekennzeichnet, dass**
eine wirksame Höhe des Aufnahmeraums (16) kleiner als der doppelte Durchmesser des aufzunehmenden Führungsdrahtes ist.

5. Einrichtung nach einem der vorhergehenden Ansprüche,
**dadurch gekennzeichnet, dass**
die Einführöffnung (28) eine zum Aufnahmeraum (16) weisende Einführschräge (29) aufweist.

6. Einrichtung nach Anspruch 1,
**dadurch gekennzeichnet, dass**
der Aufnahmeraum (16) zur Aufnahme der nebeneinander angeordneten Windungen des Wickelgutes (15) radial zur Rotationsachse (17) der Wickelgutaufnahme (12) und des Gehäuseteils (14) ausgerichtet ist.

7. Einrichtung nach Anspruch 1,
**dadurch gekennzeichnet, dass**
die Wickelgutaufnahme (12) und das Gehäuseteil (14) durch eine lösbare Rastverbindung (22) drehbar miteinander verbunden sind und vorzugsweise zumindest ein, insbesondere federnd nachgiebiges, Rastelement (23) aufweist, welches an der Wickelgutaufnahme (12) oder dem Gehäuseteil (14) angeformt ist.

8. Einrichtung nach Anspruch 1,
**dadurch gekennzeichnet, dass**
an der Wickelgutaufnahme (12) oder dem Gehäuseteil (14) ein Lueranschluss oder Spülöffnung (49) vorgesehen ist.

## Claims

1. Device for receiving an elastically deformable guide wire (15),
with a winding material receptacle (12), and
with a housing part (14) at least partially covering the winding material receptacle (12), which form a ring-shaped receptacle space (16) for receiving the medical guide wire (15),
wherein a winding material guidance (33) is provided in the winding material receptacle (12), which arranges a plurality of windings of the guide wire (15) next to each other when winding up the guide wire (15),
wherein the winding material guidance (33) is configured by a profiled surface in the receptacle space (16) and the surface (34) is made of a circumferential groove (36), which is separated by a bar (37) arranged there-between, which bar continues helically, such that the windings of the guide wire (15) are arranged next to each other in one plane in the receptacle space (16) and are wound up separated from each other,
**characterized in that**
the winding material receptacle (12) and the housing part (14) are connected pivotably relative to each other around a common rotation axis (17),
an insertion opening (28) leading to the receptacle space (16) is provided in the housing part (14),
at least one gripping surface (31), in particular a gripping recess, is provided at the outside of the winding material receptacle (12) and/or the outside of a base (18) of the housing part (14).

2. Device according to claim 1,
**characterized in that**
the circumferential groove (36) has a groove depth which corresponds to at least half the diameter of the guide wire (15) to be received.

3. Device according to claim 1,
**characterized in that**
a distance between a contact surface (38) of the housing part (14) and a front face of the circumferential groove (36) is smaller than the diameter of the smallest guide wire (15) to be received, in particular smaller than half the diameter of the smallest guide wire (15) to be received.

4. Device according to claim 1,
**characterized in that**
an effective height of the receptacle space (16) is smaller than twice the diameter of the guide wire to be received.

5. Device according to one of the preceding claims,
**characterized in that**
the insertion opening (28) has an insertion chamfer (29) pointing to the receptacle space (16).

6. Device according to claim 1,
**characterized in that**
the receptacle space (16) for receiving the winding material's (15) windings being positioned next to each other are aligned radially to the rotation axis (17) of the winding material receptacle (12) and of the housing part (14).

7. Device according to claim 1,
**characterized in that**
the winding material receptacle (12) and the housing part (14) are pivotably connected via a detachable latching connection (22), and preferably have at least one, particularly resilient latching connection (23), which is integrally formed to the winding material receptacle (12) or to the housing part (14).

8. Device according to claim 1,
**characterized in that**
a Luer-connector or rinsing opening (49) is provided at the winding material receptacle (12) or to the housing part (14).

## Revendications

1. Dispositif pour ranger un fil de guidage médical flexible (15), avec un rangement de fil enroulé (12) et une partie de boîtier (14) recouvrant au moins en partie le rangement de fil enroulé (12), qui forme un espace de rangement annulaire (16) pour ranger le fil de guidage médical (15),
dans lequel est prévu dans le rangement de fil enroulé (12) un guide d'enroulement du fil (33) qui dispose plusieurs tours du fil de guidage (15) les uns près des autres lors de l'enroulement du fil de guidage (15),
dans lequel le guide d'enroulement du fil (33) est formé par une surface profilée dans l'espace de rangement (16,) et la surface (34) étant formée à partir d'une rainure circonférentielle (36) qui est séparée par une barrette (37) disposée entre les tours, qui se poursuit en colimaçon de sorte que les tours du fil de guidage (15) sont disposés les uns à côté des autres dans le même plan dans l'espace de rangement (16) et sont enroulés séparément les uns des autres,
**caractérisé en ce que** le rangement de fil enroulé (12) et la partie de boîtier (14) sont reliés entre eux de façon à pouvoir tourner l'un par rapport à l'autre autour d'un axe de rotation commun (17), **en ce qu'**il est prévu dans la partie de boîtier (14) une ouverture d'introduction (28) donnant sur l'espace de rangement (16),
**en ce qu'**il est prévu sur un côté extérieur du rangement de fil enroulé (12) et/ou sur un côté extérieur d'une surface de base (18) de la partie de boîtier (14) au moins une surface de prise (31), en particulier un creux de prise.

2. Dispositif selon la revendication 1, **caractérisé en ce que** la rainure circonférentielle (36) présente une profondeur de rainure correspondant au moins à la moitié du diamètre du fil de guidage (15) à recevoir.

3. Dispositif selon la revendication 1, **caractérisé en ce qu'**une distance entre une surface d'appui (38) de la partie de boîtier (14) et une face frontale de la rainure circonférentielle (36) est plus petite que le fil de guidage (15) du plus petit diamètre à recevoir, en particulier plus petite que la moitié du diamètre du plus petit fil de guidage (15) à recevoir.

4. Dispositif selon la revendication 1, **caractérisé en ce qu'**une hauteur efficace de l'espace de rangement (16) est plus petite que le double du diamètre du fil de guidage à recevoir.

5. Dispositif selon l'une des revendications précédentes, **caractérisé en ce que** l'ouverture d'introduction (28) présente un biseau d'introduction (29) dirigé vers l'espace de rangement (16).

6. Dispositif selon la revendication 1, **caractérisé en ce que** l'espace de rangement (16) est orienté pour recevoir les tours juxtaposés du fil enroulé (15) dans le sens radial par rapport à l'axe de rotation (17) du rangement de fil enroulé (12) et de la partie de boîtier (14).

7. Dispositif selon la revendication 1, **caractérisé en ce que** le rangement de fil enroulé (12) et la partie de boîtier (14) sont reliés entre eux par un assemblage encliqueté (22) pouvant être défait et présentent au moins un élément d'encliquetage (23), en particulier souple et élastique, qui est formé sur le rangement de fil enroulé (12) ou sur la partie de boîtier (14).

8. Dispositif selon la revendication 1, **caractérisé en ce qu'**un raccord Luer ou une ouverture de rinçage (49) sont prévus sur le rangement de fil enroulé (12) ou la partie de boîtier (14).
